# EUROPEAN PATENT SPECIFICATION

(11) **EP 1 629 728 B1**
(45) Date of publication and mention of the grant of the patent: **26.11.2008**
(21) Application number: 05016296.5
(22) Date of filing: 27.07.2005
(51) Int. Cl.: A23L 1/30, A23L 1/0522, A61K 31/738, A61P 3/08

(54) **Use of chemically modified starch product**
Verwendung eines chemisch modifizierten Stärkeprodukts
Utilisation d'un produit à base d'amidon chimiquement modifié

(30) Priority: 29.07.2004 US 591983 P; 29.07.2004 US 591997 P; 06.07.2005 US 175456
(43) Date of publication of application: 01.03.2006
(73) Proprietor: BRUNOB II B.V., 6824 BM Arnhem (NL)
(72) Inventor: Brown, Ian, Basking Ridge New Jersey 07920 (US); Okoniewska, Monica K., Princeton New Jersey 08540 (US); Billmers, Robert L., Stockton New Jersey 08559 (US); Skorge, Robert A., Somerville New Jersey 08876 (US)
(74) Representative: Held, Stephan

(56) References cited:
- EP-A- 0 749 697
- EP-A- 1 602 376
- US-A1- 2003 045 504
- WOLF BW, BAUER LL, FAHEY GC, JR: "Effects of chemical modification on in vitro rate and extent of food starch digestion: an attempt to discover a slowly digested starch" J. AGRIC. FOOD CHEM., vol. 47, 1999, pages 4178-4183, XP002358151
- RABEN A, ANDERSEN K, KARBERG MA, HOLST JJ, ASTRUP A: "Acetylation of or B-cyclodextrin addition to potato starch: beneficial effect on glucose metabolism and appetite sensations" AM. J. CLIN. NUTR., vol. 66, 1997, pages 304-314, XP002358152
- WOO K S ET AL: "CROSS-LINKED RESISTANT STARCH: PREPARATION AND PROPERTIES" CEREAL CHEMISTRY, AACC INTERNATIONAL, ST PAUL, MN, US, vol. 79, no. 6, November 2002 (2002-11), pages 819-825, XP001132662 ISSN: 0009-0352

## Description

The present invention relates to the use of starch which is modified by a process selected from the group consisting of propylene oxide modification, octenyl succinic anhydride modification, acetylation, dextrinization, and combinations thereof for the preparation of an edible product for controlling the blood glucose level of a mammal comprising ingesting said product wherein the starch is in granular form and has an altered digestion profile such that less than 25% is digested within the first 20 minutes of ingestion, 30 to 70% is digested within 120 minutes of ingestion and at least 60 % is digested within 240 minutes of ingestion, wherein the digestion profile is measured according to the simulated digestion method of Englyst as described in the description.

The use of the starch is helpful to control and/or regulate the blood glucose level of mammals after consumption and postprandial absorption. Also useful are starches treated with heat and/or acid (dextrinization), thermal or hydrothermal (heat and moisture), or other physical processes to impart the desired digestibility. The treatment is applied at a level and type to control and/or regulate the blood glucose level of mammals when used as a food or feed source by modifying the time and rate of post-prandial absorption.

Starch is a major source of energy in the typical western diet. Refined starches (for a description of refined starches see Imberty et al. Die Starke, 43 (10), 375-84 (1991)) are mostly eaten in the cooked form, which generally provides a high and rapid rise in blood glucose, being quickly and completely digested. However, some refined starches can resist enzymatic hydrolysis in the small intestine, such that the starch is not substantially broken down until it reaches the large intestine where it is utilized by resident microorganisms (this is defined as resistant starch or RS). Englyst (Englyst, H.N; et al. Eur. J. Clin. Nutr. 46 (suppl.2):S33-S50 (1992)) defined three different categories of resistant starch related to their origin and means of resistance. A fourth type of RS was later described by Brown (Brown et al. Food Australia, 43(6), 272-75 (1995)) relating to chemically modified starches containing ethers, esters and cross-bonded starches that are resistant to enzymatic digestion.

The term available carbohydrate is defined as the total amount of carbohydrate in a food minus the amount of carbohydrate that is non-digestible. Non-digestible carbohydrates include dietary fiber, sugar alcohols and non-digestible sugars. Dietary fiber includes the group of starches defined above by Englyst and Brown (RS1 to 4). In some published examples, resistant starch is measured or quantified as dietary fiber (e.g. Chui et al. US Pat. # 5,902,410) using standard test methods (see AOAC 985.29 and 991.42) and provide little to no absorbable postprandial glucose, but are fermented in the large intestine. Furthermore, the presence of resistant starch affects the amount of available carbohydrates in the food serving in the same way as dietary fiber (e.g., cellulose, inulin, bran, psylium) affects the quantity of available carbohydrates.

Glycemic response (GR) refers to the differential effects of foods on blood glucose levels over the time period of 0 to 120 minutes (NIH Publication Number 99-3892, 1999). It is measured as the incremental area under the blood glucose response curve in an individual subject for a particular food sample on a specific day. The magnitude and duration of the glycemic response to various foods reflects the variability in the rate and extent of the digestion and absorption of glucose containing components such as starch. This method has been used to determine the magnitude of the postprandial glucose response to an individual food and also to compare (relative glycemic response) foods using the same sample or serving size. This is useful in determining the effects on blood glucose of foods as consumed by humans and animals.

As used in this application, glycemic index (GI) (Jenkins, D.J.A. et al., Am. J. Clin. Nutr. 34(3): 362-66, 1981) is defined as "the incremental area under the blood glucose response curve of a 50 g available carbohydrate portion of a test food expressed as a percent of the response to the same amount of available carbohydrate in a standard food taken by the same subject". An arbitrary value of 100 has been assigned for the standard food, which can either be 50 g of glucose or 50 g of white bread.

The GI seeks to quantify the interactions of various ingredients in food and the role they play in how a carbohydrate source is digested and the glucose absorbed. By requiring a specified amount of available carbohydrate (50g) in the test food, a larger (sometime much larger) portion of the test food must be consumed. Alternatively stated, foods rich in fats, protein or dietary fiber would necessitate a larger serving size in order to ingest the required 50g of available carbohydrate.

As the food is ingested, the amount of glucose in the blood is subject to two basic mechanisms. The first is the rate of absorption into the blood stream of glucose as the food is digested. The second mechanism is the rate of absorption of the glucose from the bloodstream into the body tissue. Although this is a simplified view of these two mechanisms, one skilled in the art would recognize the complex and multifaceted nature of the mechanisms, reactions and processes involved. In normal healthy individuals, the body has mechanisms for regulating the blood glucose levels within certain specific ranges (fasting plasma glucose levels of 3.9 to 6.1 mmol/L as specified by the American Diabetes Association, Diabetes Care, 24(suppl), 1-9 (2001)). For example, increases in blood glucose levels stimulate the production of insulin, which amongst other functions facilitates the absorption of glucose into the tissue, but also exerts major functions in the metabolism of fats and proteins. Therefore, foods that cause an acute elevation in blood glucose concentration, have been shown to produce a rapid (but offset) rise in serum insulin levels, which leads to the uptake, storage and use of glucose by the muscle cells, adipose tissue and the liver, consequently balancing the blood glucose concentration in the "normal" range.

Glucose that is absorbed into the tissue can be converted to glycogen as a means of storage for the muscles. Glycogen is used in times of physical activity and replenished in times of rest. Carbohydrate (carb) loading is a process athletes use to increase the store of energy (in the form of glycogen) in the muscles before an athletic event. It is "a strategy in which changes to training and nutrition can maximize muscle glycogen stores prior to an endurance competition" (Michelle Minehan, AIS Sports Nutrition Program, 2003). Glycogen can also be transported from the muscle to the bloodstream to increase blood glucose levels if they fall below certain levels.

A number of conditions are associated with over/under production of insulin or the reaction of cells in the body to the actions normally initiated by insulin. Insulin resistance (IR) is the condition in which the body tissue becomes less receptive to insulin and requires higher levels to achieve the same physiological effect. The principal effects of IR have been identified as decreased utilization of glucose by the body cells, resulting in increased mobilization of fats for the fat storage areas, and depletion of protein in the tissues of the body (Guyton, A.C., "Textbook of Medical Physiology (7th Ed.), W.B. Saunders Company: Philadelphia, Pa. 923-36). Other conditions arising from the over/under production of insulin include hypoglycemia, hyperglycemia, impaired glucose regulation, insulin resistance syndrome, hyperinsulinemia, dyslipidemia, dysfibrinolysis, metabolic syndrome, syndrome X and diabetes mellitus (type II also known as non-insulin depended diabetes mellitus (NIDDM) and the physiological conditions that may arise such as cardiovascular disease, retinopathy, nephropathy, peripheral neuropathy and sexual dysfunction.

Another affect often associated with acute elevation and rapid swings in blood glucose levels is the inability to control and maintain body weight. Insulin, which plays many roles in the body, is also active in the conversion of glucose to fats (Anfinsen et al. US Pat# 2004/0043106). Insulin resistance, necessitating higher levels of serum insulin, is thought to be a cause of weight gain as the increased insulin levels facilitate unnecessary fat storage. Experts have long recommended eating many small meals over the course of a day to attempt to regulate blood glucose (and the corresponding energy supply) at a constant, uniform level. Additionally, rapidly falling blood glucose levels (which normally happens after an acute elevation) have been shown to trigger a stimulation of appetite (hunger) in healthy adult humans. Alternatively, research indicates that glucose release over an extended time period leads to specific benefits which may include increased satiety for longer time periods (weight management such as weight loss and long term weight stabilization), sustained energy release (enhanced athletic performance including training), and improvements in mental concentration and memory.

A starch, or starch-rich material, which could provide glucose to the blood over an extended time would serve to maintain normal/healthy blood glucose levels (i.e. normoglycemia) and reduce/eliminate rapid changes in blood glucose level. It would potentially be an excellent carbohydrate source in the prevention and treatment of any of the conditions discussed above. Healthy individuals wishing to control glucose release or regulate the energy release from foods as well as the prevention or treatment of many diseases associated with irregularities in blood glucose and insulin concentrations could utilize foods containing these starches.

EP 1 602 376 A1 relates to a preventive/remedy for obesity, which has a hydroxypropylated starch as its active ingredient. Provided are materials for foods, drugs, etc. which can exhibit an effect of preventing/lessening the onset of various lifestyle related diseases.

Wolf et al., J. Agric. Food Chem., 1999, 47, 4178-4183, relates to the effects of chemical modification on in vitro rate and extent of food starch digestion. It discloses an attempt to discover a slowly digestible starch. Wolf et al. disclose that differences in glycemic and insulinemic responses to dietary starch are directly related to the rate of starch digestion. It is speculated that chemical modification of starch may allow for the production of a slowly digested starch that could be used for the treatment of certain medical modalities. The extent of starch digestion was significantly reduced by dextrinization, etherification and oxidation. However the rate of starch digestion was not significantly affected by chemical modification.

Raben et al. , Am. J. Clin. Nutr., 1997, 66, 304-314 describes the acetylation of or beta-cyclodextrine addition to potato starch. Beneficial effects on glucose metabolism and appetite sensations are discussed. As a result of this study, the minor modification insulinemic (1 to 2%) of native potato starch improved the glycemia, insulinemic and satiating properties of a meal. This is especially true for the beta-cyclodextrine-enriched starch.

Woo et al., Cereal Chem., 79(6), 819-825, relates to resistant starches, their preparation and properties. The resistant starches have been prepared by phosphorylation of various starch base materials.

Surprisingly, it has now been discovered that chemically modified starch may be used to control and/or regulate the blood glucose level of mammals after consumption and postprandial absorption. The treatment is applied at a level and type to control and/or regulate the blood glucose level of mammals when used as a food or feed source by modifying the time and rate of post-prandial absorption. It has further been discovered that such chemically modified starches, when properly formulated into foods or used as supplements, may be used to provide the consumer with a controlled and/or regulated supply of glucose to the blood over an extended time period.

The present invention relates to the use of starch which is modified by a process selected from the group consisting of propylene oxide modification, octenyl succinic anhydride modification, acetylation, dextrinization, and combinations thereof for the preparation of an edible product for controlling the blood glucose level of a mammal comprising ingesting said product wherein the starch is in granular form and has an altered digestion profile such that less than 25% is digested within the first 20 minutes of ingestion, 30 to 70% is digested within 120 minutes of ingestion and at least 60 % is digested within 240 minutes of ingestion, wherein the digestion profile is measured according to the simulated digestion method of Englyst as described in the description.

The use of the starch is helpful to control and/or regulate the blood glucose level of mammals after consumption and postprandial absorption. Such chemically modified starches, capable of reducing the initial acute elevation of blood glucose, and when properly formulated into foods, may be used to provide the consumer with controlled/regulated glucose over an extended time period and assist in providing normal/healthy blood glucose levels, even in individuals who may/could develop insulin resistance.

As used herein, the term chemically modified is intended to mean any chemical modification known in the art of starch, including without limitation starch treated with acetic anhydride (AA), propylene oxide (PO), succinic anhydride (SA), octenyl succinic anhydride (OSA), crosslinking reagents such as sodium trimetaphosphate (STMP), phosphorus oxychloride (POCl₃), epichlorohydrin, adipic acetic anhydride, phosphorylating reagents such as sodium tripolyphosphate (STPP) or ortho phosphates, oxidizing reagents such as sodium hypochlorite or peroxide or other food approved starch modifying reagents, enzymes or physical processes such as heat/acid (dextrinization) thermal or hydrothermal (heat and moisture), or other physical processes and combinations thereof in order to alter the digestibility and rate of postprandial absorption.

Granular, as used herein, is intended to mean non-gelatinized or dispersed by any chemical or physical process. Granular starches can be determined using microscopy by the presence of birefringence (Maltese cross) under polarized light. Granular starches are also not significantly soluble in water below their gelatinization temperature. Non-granular starches are those that have been treated or processed to be readily soluble in water (CWS) below their gelatinization temperature (typically about 65°C). Some starches can be processed to become soluble and then are allowed to retrograde so as to form particles (crystallites) that are no longed soluble in water below 100°C, but are also not granular. In an embodiment of this invention, the granular form of starch was used.

Most researchers and publications have chosen two points in time to measure the digestibility of carbohydrates. These points are at 20 and 120 minutes, but do not accurately reflect the breakdown to, or absorption of, glucose in the stomach and the entire length of the small intestine. For purposes of this application, digestion and absorption of various samples have been measured at 20, 120 and 240 minutes to better relate to the true physiological effects these samples will have in the mammalian digestive system.

As used herein, the term rapidly digestible starch is intended to mean a starch or portions thereof which are fully absorbed within the first 20 minutes after ingestion.

As used herein, the term resistant starch has been defined as "the sum of starch and products of starch digestion not absorbed in the small intestine of healthy individuals" (EJCN, 1992, 46 suppl.2 S1).

The term slowly digestible starch is intended to mean a starch, or the fraction thereof, which is neither rapidly digestible starch nor resistant starch. Alternatively stated, slowly digestible starch is any starch (granular, non-granular, or retrograded) that releases its glucose to the mammalian body over the entire length of the stomach and small intestine (typically between 20 minutes and 240 minutes in humans). For a similar and more complete description of these starches see Englyst et al., European Journal of Clinical Nutrition, 1992, 46, S33-S50. (Note: Englyst describes slowly digestible starches as those that release their glucose between 20 and 120 minutes as opposed to between 20 and 240 minutes.)

As used herein, anhydrous borax fluidity (ABF) is defined as the ratio of the amount of water to the amount of anhydrous dextrin when the latter is cooked for 5 minutes at 90 °C with 15% borax based on the weight of the dextrin, so as to provide a dispersion having a viscosity, when cooled to 25°C of 70 cps. Anhydrous borax fluidity is a term known in the art.

As used herein, water fluidity (WF) is intended to mean a starch measurement using a Thomas Rotational Shear-type Viscometer (commercially available from Arthur A. Thomas CO., Philadelphia, PA), standardized at 30°C with a standard oil having a viscosity of 24.73 cps, which oil requires 23.12±0.05 sec for 100 revolutions. Accurate and reproducible measurements of water fluidity are obtained by determining the time which elapses for 100 revolutions at different solids levels depending on the starch's degree of conversion: as conversion increases, the viscosity decreases. Water fluidity is a term known in the art.

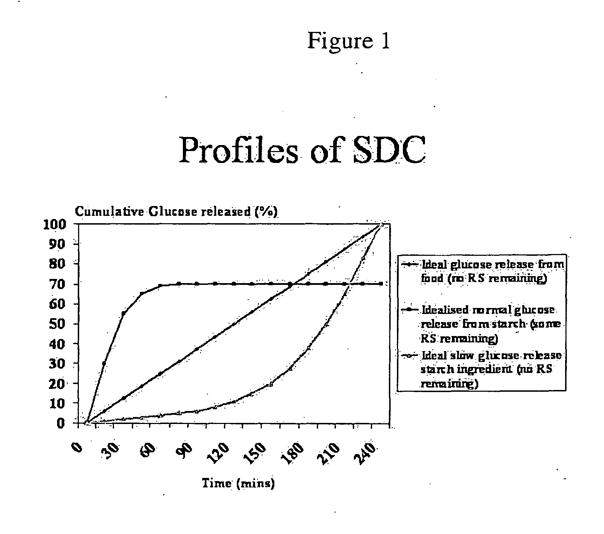

Figure 1 shows the ideal slow glucose release compared to that of normal starches, and the ideal glucose release from foods containing such starches.
Figure 2 depicts the actual glucose release of uncooked corn starches crosslinked to various levels with STPP/STMP.

The chemically modified starches, which when properly formulated into foods or taken as a supplement, may be used to provide the consumer with more constant blood glucose (prevent/minimize acute elevation) levels over an extended time period (corresponding to the time the material is in the stomach/small intestine) than would be possible with other types of starches. Such starches and foods containing these starches will help the consumer regulate and maintain normal and healthy blood glucose levels.

Starch, as used herein, is intended to include all starches, flours, grits and other starch containing materials derived from tubers, grain, legumes and seeds or any other native source, any of which may be suitable for use herein. A native starch as used herein, is one as it is found in nature. Also suitable are starches derived from a plant obtained by standard breeding techniques including crossbreeding, translocation, inversion, transformation or any other method of gene or chromosome engineering to include variations thereof which are typically referred to as genetically modified organisms (GMO). In addition, starch derived from a plant grown from artificial mutations (including those from chemical mutagens) and variations of the above generic composition, which may be produced by known standard methods of mutation breeding, are also suitable herein.

Typical sources for the starches are cereals, tubers, roots, legumes and fruits. The native source can be corn (maize), pea, potato, sweet potato, banana, barley, wheat, rice, oat, sago, amaranth, tapioca (cassava), arrowroot, canna, triticale, and sorghum as well as waxy or high amylose varieties thereof. As used herein, the term "waxy" or "low amylose" is intended to include a starch containing no more than about 10%, particularly no more than about 5%, most particularly no more than about 2%, by weight amylose. Also used herein, the term "high amylose" is intended to include a starch containing at least about 40%, particularly at least about 70%, most particularly at least about 80%, by weight amylose. The invention embodied within relates to all starches regardless of amylose content and is intended to include all starch sources, including those which are natural, genetically altered or obtained from hybrid breeding.

The starch is chemically modified using methods known in the art. In one embodiment, the starch is treated with acetic anhydride (AA), propylene oxide (PO), succinic anhydride (SA), octenyl succinic anhydride (OSA), crosslinking reagents such as STMP, POCl₃, epichlorohydrin or adipic acetic anhydride, phosphorylating reagents such as sodium tripolyphosphate (STPP) or ortho phosphates, oxidizing reagents such as sodium hypochlorite or peroxide or other food approved starch modifying reagents, enzymes or physical processes such as heat/acid (dextrinization) thermal or hydrothermal (heat and moisture), or other physical processes and combinations thereof. Such chemical modifications are known in the art and are described for example in Modified Starches: Properties and Uses, Ed. Wurzburg, CRC Press, Inc., Florida (1986). It is also possible to use enzymes, alone or in combination with other chemical treatments, to obtain starches of this invention. Enzymes are classified by function such as those that alter molecular weight and others that change chemical or architectural structure. Such enzymes include, but not limited to, alpha amylase, glucoamylase, pullulanase, beta amylase, isomerases, invertases, and transamidases. If the starch is modified with STMP and/or STPP, the starch base must be a high amylose starch.

One skilled in the art would recognize that by varying the reaction conditions and reagents it may be possible to vary the level of substitution and possibly the location within the starch molecule. The mechanisms for digestion and absorption depend upon various factors, including starch type, amylose content and granular composition/conformation as well as reagent type, and reaction conditions. The rate of digestion is also dependent on the way or manner the food is prepared and the reaction of the individual to such foods, including variations in each individual's biochemistry and physiology. The mechanism by which starch is processed in the body is well known in the art.

The amount of chemical modification may be varied to get the desired digestion profile. Chemical modification includes, without limitation, any reagent known in the art capable of producing a starch ether or ester which has been or will be approved by the appropriate regulatory agency for consumption. Examples of such reagents are, but not limited to, acetic anhydride, propylene oxide, succinic anhydride, octenyl succinic anhydride, crosslinking reagents such as STMP, POCl₃, epichlorohydrin or adipic acetic anhydride and phosphorylating reagents such as sodium tripolyphosphate or sodium metaphosphate and combination of these.

Additionally, reagents and processes capable of altering the chemical structure, conformation or crystallinity of the starch to render it less susceptible to digestion in the body are also included in the invention. Such reagents include oxidative reagents and processes, the action of heat and/or acid such as dextrinization, the action of enzymes and combinations of these with or without chemical modifications.

Other modification that may not affect the digestion profile, but may provide desirable textural and/or physical properties are also included in the scope of this application. The additional modification may be accomplished before or after the chemical modification using for example thermal inhibition or chemical cross-linking to toughen the starch and provide shear resistance during processing. It would be within the knowledge of the skilled artisan as to what combinations are possible and in what order such modification may be accomplished. Additional modifications may include certain types of molecular weight reduction (for viscosity control) such as acid conversion or enzyme degradation.

Modifications as described above are typically accomplished in aqueous media with some form of pH control or pH adjustment. A skilled practitioner would readily appreciate the variety of materials and equipment for carrying out these reactions. For a review of these reaction conditions see Modified Starches: Properties and Uses, Ed. Wurzburg, CRC Press, Inc., Florida (1986), chapter 4. Other reaction media and conditions may be utilized and will provide materials under the scope of the invention. These include, but are not limited to, dry heat reactions, solvent reactions, supercritical fluid reactions and gaseous conditions.

The starch may be modified by physical means. Physical modification includes by shearing, hydrothermal or thermal-inhibition, for example by the process described in U.S. Patent No. 5,725,676.

The starch may be modified by enzymatic means. Enzymatic modification includes by exo- and/or endo-enzymes, including without limitation, by alpha-amylase, beta-amylase, glucoamylase, maltogenase, pullulanase and isoamylase or any combination of the above.

These starches may be modified in the granular state or after gelatinization using techniques known in the art. Such techniques include those disclosed for example in U.S. Patent Nos. 4,465,702, 5,037,929, 5,131,953, and 5,149,799. Also see, Chapter XXII- "Production and Use of Pregelatinized Starch", Starch: Chemistry and Technology, Vol. III- Industrial Aspects, R.L. Whistler and E.F. Paschall, Editors, Academic Press, New York 1967.

The starches of this invention may be converted, such as fluidity or thin-boiling starches prepared by oxidation, acid hydrolysis, enzyme hydrolysis, heat and or acid dextrinization. These processes are well known in the art.

The starch may be purified by any method known in the art to remove starch off flavors, colors, or sanitize microbial contamination to insure food safety or other undesirable components that are native to the starch or created during processing. Suitable purification processes for treating starches are disclosed in the family of patents represented by EP 554 818 (Kasica, et al.). Alkali washing techniques are also useful and described in the family of patents represented by U.S. 4,477,480 (Seidel) and 5,187,272 (Bertalan et al.). The starch may be purified by enzymatic removal of proteins. Reaction impurities and by-products may be removed by dialysis, filtration, centrifugation or any other method known in the art for isolating and concentrating starch compositions. The starch may be washed using techniques known in the art to remove soluble low molecular weight fractions, such as mono- and di-saccharides and/or oligosaccharides.

In one embodiment, the starch is modified by a process selected from the group consisting of propylene oxidation in the range of 3-10% bound, OSA modification in the range of 1.5-3.0% bound, acetylation in the range of 0.5 to 3.0% bound, dextrinization to a canary or white dextrin in the range of less than 10 ABF, and combinations thereof. In another embodiment, the starch is further modified by a process selected from the group consisting of acid or enzyme conversion to a water fluidity of 20-85, hypochloride treatment at a level of 0.4-5.0%, adipic acetic treatment at a level of 0.1 to 2.0%, phosphorus oxychloride treatment at a level of 0.001 to 0.5% treatment, and combinations thereof. In yet another embodiment, the starch is treated with sodium trimetaphosphate and/or sodium tripolyphosphate at a level of 0.1 to 0.35% added bound phosphate and hypochloride treatment at a level of 0.3 to 1.0%.

The resultant starch is typically adjusted to the desired pH according to its intended end use. In general, the pH is adjusted to 3.0 to about 6.0. In one embodiment, the pH is adjusted to 3.5 to about 4.5, using techniques known in the art.

The starch may be recovered using methods known in the art, particularly by filtration or by drying, including spray drying, freeze drying, flash drying or air drying. In the alternative, the starch may be used in the liquid (aqueous) form.

The resultant starch has an altered digestion profile, such that less than 25% is digested within the first 20 minutes, in another embodiment less than 20% is digested, and in yet another embodiment less than 10%, is digested within the first 20 minutes of ingestion.

Further, the resultant starch is 30 to 70% digested within 120 minutes of ingestion. In one embodiment, the starch is at least 40-60% digested within 120 minutes of ingestion and in another embodiment, at least 45-55% digested within 120 minutes.

In addition, the resultant starch is at least 60% digested within 240 minutes of ingestion. In one embodiment, the starch is at least 70% digested within 240 minutes of ingestion and in another embodiment, at least 80% digested within 240 minutes and in yet another embodiment, at least 90% digested within 240 minutes.

One skilled in the art would be able to alter the glucose release. For example if glucose release is too high the chemical modifications which will help reduce glucose release to the desired level include without limitation higher crosslinking level using STMP, STPP, phosphorus oxychloride, and/or adipic-acetic acid; and/or increased substitution with propylene oxide, OSA, or acetylation. If glucose release is too low, chemical modifications which will help increase glucose release include without limitation lower crosslinking level using STMP, STPP, phosphorus oxychloride and/or adipic-acetic acid; and/or hypochloride treatment, manganese oxidation conversion, and or other oxidation treatments. Combinations of chemistries have to be adjusted to a starch base and consider the effect of complementary treatments.

It would be apparent to one skilled in the art that cooking a starch will affect the digestibility and rate of absorption of the glucose into the blood stream. For a review of the effect of cooking see Brown, M.A., et al. British Journal of Nutrition, 90, 823-27 (2003).

In a recent patent application, Brown et al., US 2003/0045504A1 published March 6, 2003 shows the relationship between resistant starch and other components in the foods (such as various lipids) and their affect on the digestibility, Gl, glucose response (GR) and blood glucose levels after ingestion of such foods containing resistant starch.

Starch is rarely consumed on its own, but is typically consumed as an ingredient in a food product. This food product may be manipulated to result in desired glucose release curves. In one embodiment, the food is manipulated to provide a substantially zero order glucose release curve, to provide an essentially constant and sustained glucose release rate.

Starch or starch rich materials (e.g., flour or grits) may be consumed in its raw state, but is typically consumed after cooking and/or other processing. Therefore, those starches are useful which, when added to food and processed, have the advantage of changing the glucose release curve. In one embodiment, the food containing the processed starch provides a substantially zero order glucose release curve, to provide an essentially constant and sustained glucose release rate. Such foods are modeled by the methods described in the Examples section, *infra.*

The chemically modified starch does not produce a large rapid increase in blood glucose levels typical of high glycemic index starches, such as most native starches. Instead, these modified starches provide a more moderate increase above the baseline which is sustained for a longer time period. It is also process tolerant in that there is no large and rapid increase in blood glucose levels after ingestion of food containing the starch and the glucose release from the prepared and/or processed food is substantially constant.

The chemically modified starches described may be used in a variety of edible products including, but not limited to: baked goods, including crackers, breads, muffins, bagels, biscuits, cookies, pie crusts, and cakes; cereal, bars, pizza, pasta, dressings, including pourable dressings and spoonable dressings; pie fillings, including fruit and cream fillings; sauces, including white sauces and dairy-based sauces such as cheese sauces; gravies; lite syrups; puddings; custards; yogurts; sour creams; beverages, including dairy-based beverages; glazes; condiments; confectioneries and gums; and soups.

Edible products also are intended to include nutritional foods and beverages, including dietary supplements, diabetic products, products for sustained energy release such as sports drinks, nutritional bars and energy bars.

The chemically modified starch may be also used in a variety of animal feed products, weaning formulations affording desirable growth and development of the post weaned animal, pharmaceutical formulations, nutriceuticals, over the counter (OTC) preparations, tablets, capsules and other known drug delivery vehicles for human and/or animal consumption and/or any other applications that can benefit from constant release of glucose from the formulation.

The chemically modified starches may be added in any amount desired or necessary to obtain the functionality of the composition. In one embodiment, the starch may be added in an amount of from 0.01% to 99% by weight of the composition. In another embodiment, the starch is added in an amount of from 1 to 50%, by weight of the composition. The starch may be added to the food or beverage in the same manner as any other starch, typically by mixing directly into the product or adding it in the form of a solution.

Edible products may be formulated using the modified starch to provide a substantially zero order glucose release rate. Such products may provide the consumer with glucose over an extended time period and more constant blood glucose levels.

Products which control and/or regulate the rate and magnitude of glucose adsorption may increase satiety for longer time periods, and thus be useful in weight management. They may also provide sustained energy release, and thus enhance athletic performance including training, and improvements in concentration maintenance and memory.

The products may also provide pharmaceutical benefits, including reducing the risk of developing diabetes, treating obesity such as weight loss or weight management, and preventing or treating hyperglycemia, insulin resistance, hyperinsulinemia, dyslipidemia, and dysfibrinolysis.

The following examples are presented to further illustrate and explain the present invention and should not be taken as limiting in any regard. All percents used are on a weight/weight basis.

The following test procedures are used throughout the examples:

### Simulated Digestion - (Englyst et al, European Journal of Clinical Nutrition, 1992 46.S33-S50)

Food samples are ground/minced as if masticated. Powder starch samples are screened to a particle size of 250 microns or less. 500-600 mg ± 0.1 mg of sample is weighed and added to the sample tube. 10 ml of a pepsin (0.5%), guar gum (0.5%), and HCl (0.05 M) solution are added to each tube.

Blank and glucose standard tubes are prepared. The blank is 20 ml of a buffer containing 0.25 M sodium acetate and 0.02% calcium chloride. Glucose standards are prepared by mixing 10 ml sodium acetate buffer (described above) and 10ml of 50 mg/ml glucose solution. Standards are prepared in duplicate.

The enzyme mix is prepared by adding 18 g of porcine pancreatin (Sigma P-7545) to 120 ml of deionized water, mixing well, then centrifuging at 3000g for 10 minutes. The supernatant is collected and 48mg of dry invertase (Sigma I-4504) and 0.5 ml AMG E (Novo Nordisk) are added.

The sample tubes are pre-incubated at 37°C for 30 min, then removed from the bath and 10 ml of sodium acetate buffer is added along with glass balls/marbles (to aid in physical breakdown of the sample during shaking).

5 ml of the enzyme mixture is added to the samples, blank, and standards. The tubes are shaken horizontally in a 37°C waterbath at approximately 180 strokes/min. Time "zero" represents the first addition of the enzyme mixture to the first tube.

After 20, 120, and 240 minutes, 0.5-ml aliquots are removed from the incubating samples and each placed into a separate tube of 20ml 66% ethanol (to stop the reaction). After 1 hour, an aliquot is centrifuged at 3000g for 10 minutes.

The glucose concentration in each tube is measured using the glucose oxidase/peroxidase method (Megazyme *Glucose Assay Procedure* GLC9/96). This is a colorimetric procedure.

The degree of starch digestion is determined by calculating the glucose concentration against the glucose standards, using a conversion factor of 0.9. Results are given as "% starch digested" (dry weight basis) after 20, 120, and 240 minutes.

Every sample analysis batch includes a reference sample of uncooked cornstarch. The accepted % digestion values for cornstarch are listed in Table I, below:

**Table I**

| Time (minutes) | 20 | 120 | 240 |
|---|---|---|---|
| Sample 1(control)¹ | 18±4 | 80±4 | 90±4 |

| | | | |
|---|---|---|---|
| ¹Melogel® starch, cornstarch commercially available from National Starch and Chemical Company, Bridgewater, NJ, USA. | | | |

### Bound Phosphorus Analysis

Prepare 1.7% slurry of starch in 5% EDTA solution and stir for 5min and filter. Wash the sample on the filter with 200 ml of deionized water four times. Dry sample at room temperature. Prepare quantitatively 3% starch slurry in 4N HCl, add boiling stones, and boil the sample for 7min, cool to room temperature, quantitatively dilute with deionized water, centrifuge to remove any possible particulate. The sample is then analyzed by Inductively Coupled Plasma Spectrometry (ICP) for phosphorus using standard analytical procedures to obtain total bound phosphorus. Added bound phosphorus is determined by subtracting total bound phosphorus of the unmodified starch from that of the modified starch.

### Model Cookie / Biscuit Food System

Measure moisture of experimental starch gravimetrically.
Calculate amount of additional water required to adjust the starch to a moisture content of 25% (w/w) which is a typical moisture level for cookie and biscuit dough.
Weigh 50g of starch into a mixing bowl of a Sunbeam Mixmaster, lower mixing blades into a bowl and turn the mixer on to a 'fold' position.
Begin addition of pre-calculated amount of water by spraying the water onto the starch while mixing to ensure even moisture distribution. Complete water addition in 5 min.; continue mixing on 'fold' setting until starch does not stick to walls of the mixing bowl. The total mixing time is 8-10min.
Transfer 50g of the hydrated starch into an aluminum tin (145mm x 120mm x 50mm) and spread evenly to cover the entire bottom of the pan.
Preheat an oven to 190°C.
Bake the hydrated starch at 190°C for 20 min.
Take the starch out from the oven, place immediately in 4oz (118.3 ml) plastic jar and close the lid.
Cool the starch to room temperature and determine moisture of baked starch gravimetrically. The moisture content of the baked starch should be in a 5-8% (w/w) range which is typical for cookies and biscuits.
Test glucose release from starch immediately or store it in an air-tight container for testing the following day.

### Example 1 - Preparation of Chemically Modified Starches

The following modifications are well-known in the art and the procedures are meant as guidance to the skilled artisan. Reagent amounts and bases may be changed to achieve different modification levels.
a) *Propylene oxide modification -* 4 g of solid sodium hydroxide are dissolved into 750 g of tap water at 23°C and mixed until completely dissolved. 50 g of sodium sulfate is then added to the water and mixed until dissolved. The tapioca starch is then added quickly to the stirring aqueous mixture and mixed until uniform. Various levels of propylene oxide are added to the starch slurry and mixed for 1 to 2 minutes. The slurry is then transferred into a 2L plastic bottle and sealed. The bottle and contents are then placed into a preheated mixing cabinet set to 40°C and agitated for 18 hours. After the reaction is complete, the slurry is adjusted to pH 3 with dilute sulfuric acid and then allowed to mix for 30 minutes. The pH is then adjusted to between 5.5 and 6.0 with dilute sodium hydroxide solution. The starch is recovered by filtration and the starch cake is washed with water (3 x 250 ml), spread out on the bench top and allowed to air dry. The example is repeated using sago starch.
b) *Octenyl succinic anhydride modification -* A total of 500 grams of waxy maize starch was placed in a 2L plastic beaker and slurried in 750 ml tap water. The slurry was mixed with an overhead stirrer while the pH was adjusted to 7.5 using 3% sodium hydroxide. The agitation of the reaction was continued while 3 aliquots of 5 grams (for a total of 15 grams) of octenylsuccinic anhydride (OSA) were added at thirty minute increments. The pH was maintained at 7.5 by addition of 3% sodium hydroxide. The reaction is allow to stir until the consumption of caustic stops (less than 1 mL in 10 minutes). The starch was then filtered through Waltman #1 paper and washed with an additional 750 ml of tap water. The starch was then reslurried in 500 ml water and the pH adjusted to 5.5 with 3:1 hydrochloric acid. The slurry was again filtered, washed with an additional 750 ml water, and air dried to less than 15% moisture to produce an OSA starch. The example was repeated using a high amylose (~70%) corn starch.
c) *Acetylated -* A total of 500 grams of waxy maize starch was placed in a 2L plastic beaker and slurried in 750 ml tap water. The beaker was equipped with an overhead stirrer and pH monitor capable of automatically adding a 3% sodium hydroxide solution to maintain a predetermined set point. The pH controller was set at 8.0 and the slurry adjusted to a pH of about 7.8. A dropping funnel was charged with 15 grams of acetic anhydride and set to deliver the full charge over approximately 1 hour while the pH was held at 8.0 with good agitation. After the addition of the anhydride was complete the reaction was allowed to continue for an additional 5 minutes at pH. The slurry was then filtered through Whatman #1 paper and washed with 3 x 500 mLs of tap water. The resulting cake is allowed to air dry to less than 15% moisture and recovered to afford the starch acetate. The example was repeated using tapoca starch.
d) *STMPlSTPP modification with bleaching (not according to the invention)-* 3,300 ml of tap water was measured into a reaction vessel. 110g Na₂SO₄ were added with agitation and stirred until dissolved. With good agitation, 2,200g high amylose (~70%) corn starch were added and then 3% NaOH was added drop-wise to the slurry as needed to reach 40ml alkalinity (733g NaOH for 44.14ml alkalinity). The slurry was stirred 1 hr and the pH was recorded (pH 11.71). The temperature was adjusted to 42°C. 220g of a 99/1 STMP/STPP blend was added and allowed to react for 17 hours. The pH was maintained with a controller and 3% NaOH (556.6g consumed). The final pH and temperature were recorded (pH 11.19 and 42°C). The pH was adjusted to 5.5 with 3:1 HCI (pH 5.49 using 285.38g HCI). The resultant starch cake was filtered and washed twice with 3,300 ml tap water. 500g of the starch was then slurried in water at 40% solids and placed in a 2L plastic beaker and slurried in 750 ml tap water. The beaker is equipped with an overhead stirrer and placed in a constant temperature bath pre-warmed to 40° and the pH is adjusted to between 10.8 and 11.2 with 3% sodium hydroxide. A total of 4.0 grams of sodium hypochlorite is added and the pH checked to confirm 10.8 - 11.2. The reaction is allowed to stir for two hours at 40°C. After two hours the slurry is adjusted to a negative KI test with a 5% Sodium meta-bisulfite solution. The starch slurry is then pH adjusted to 5.5 with dilute HCl and filtered through Whatman #1 paper and washed with an additional 750 mL of tap water. The wet cake is allowed to air dry to less than 15% moisture to afford the oxidized starch product.

### Example 2 - Preparation of Crosslinked Starches

*Sample 1* - control corn starch; Melogel® starch, commercially available from National Starch and Chemical Company, Bridgewater, NJ, USA
*Sample 2 (not according to the invention)-* 3,000 ml of tap water were measured into a reaction vessel. 100g Na₂SO₄ were added with agitation and stirred until dissolved. With good agitation, 2,000g corn starch were added and then 3% NaOH was added drop-wise to the slurry as needed to reach 40ml alkalinity (actual 667g NaOH for 44.00ml alkalinity). The slurry was stirred 1 hr and the pH was recorded (pH 11.68). The temperature was adjusted to 42°C. 160g of a 99/1 STMP/STP blend was added and allowed to react for 4 hours. The final pH and temperature were recorded (pH 11.02 and 42°C). The pH was adjusted to 5.5 with 3:1 HCl (pH 5.47 using 164.99g HCl). The resultant starch case was filtered and washed twice with 3,000 ml tap water. The cake was crumbled and air dried.
*Sample 3 (not according to the invention)-* 3,000 ml of tap water was measured into a reaction vessel. 100g Na₂SO₄ were added with agitation and stirred until dissolved. With good agitation, 2,000g corn starch were added and then 3% NaOH was added drop-wise to the slurry as needed to reach 40ml alkalinity (667g NaOH for 44.00ml alkalinity). The slurry was stirred 1 hr and the pH was recorded (pH 11.69). The temperature was adjusted to 42°C. 160g of a 99/1 STMP/STP blend was added and allowed to react for 17 hours. The final pH and temperature were recorded (pH 11.32 and 42°C). The pH was adjusted to 5.5 with 3:1 HCI (pH 5.57 using 146.88g HCI). The resultant starch case was filtered and washed twice with 3,000 ml tap water. The cake was crumbled and air dried.
*Sample 4 (not according to the invention)-* 3,300 ml of tap water was measured into a reaction vessel. 110g Na₂SO₄ were added with agitation and stirred until dissolved. With good agitation, 2,200g corn starch were added and then 3% NaOH was added drop-wise to the slurry as needed to reach 40ml alkalinity (733g NaOH for 44.14ml alkalinity). The slurry was stirred 1 hr and the pH was recorded (pH 11.71). The temperature was adjusted to 42°C. 220g of a 99/1 STMP/STP blend was added and allowed to react for 17 hours. The pH was maintained with a controller and 3% NaOH (556.6g consumed). The final pH and temperature were recorded (pH 11.19 and 42°C). The pH was adjusted to 5.5 with 3:1 HCI (pH 5.49 using 285.38g HCI). The resultant starch case was filtered and washed twice with 3,300 ml tap water. The cake was crumbled and air dried.
*Sample 5 (not according to the invention)-* 2,500 pounds (1134kg) of tap water were measured into a reaction vessel. 100 lbs (45.4kg) Na₂SO₄ were added with agitation and stirred until dissolved. With good agitation, 2,000 lbs (907.2kg) of corn starch were added. Then 3% NaOH was added at 4lbs/minute (1.8kg/minute) to the starch slurry as needed to reach 40ml alkalinity (about 600 lbs (272.2kg) NaOH for 46 ml alkalinity). The mixture was stirred for 1 hr and the pH recorded (pH 11.6). Temperature was adjusted to 108°F (42°C). 200 lbs (90.7kg) of a 99/1 STMP/STP blend were added and reacted for 17 hours. The final pH and temperature were recorded (pH 11.4 and 108 °F (42°C)). pH was adjusted to 5.5 with 3:1 HCl as needed (pH 5.4 using 75 lbs. HCl (34kg)). The starch was washed and centrifuged on a Merco centrifuge and flash dried.
*Samples 8, 9, 11, 13, 14, 15 and 16 (not according to the invention)* were prepared by the same procedure as sample 3. The amount of 99/1 STMP/STPP blend was adjusted to results in a desired bound phosphorus level.
*Samples 23 and* 26: *POCI₃ modification (not according to the invention)-* 750ml of water was measured into reaction vessel. 2.5g of NaCl were added with agitation and stirred until dissolved. 500 g of hydroxypropylated starch were added to the salt solution. 3% NaOH was added drop-wise to the slurry with strong agitation as needed to reach pH 11-11.5. The slurry was stirred 1 hr and the pH was recorded (pH 11.5). 0.02-0.2g of POCl3 was added and allowed to react for 30min while stirring at room temperature. The pH was adjusted to 5.5 with 3:1 HCl. The resultant starch cake was filtered and washed twice with 750 ml tap water. The cake was crumbled and air dried.

The amount of bound phosphorus and the amount of glucose released were determined for each of the uncooked starch samples. The results are listed in Table II, below.

**Table II**

| Starch Base | Sample ID | STMP/STPP | Bound Phosphorus | Glucose Released O/T (%) | | |
|---|---|---|---|---|---|---|
| | | (% on starch) | (%) | 20min | 120min | 240min |
| Dent corn | 1 | Native | 0.04 | 17 | 75 | 85 |
| Dent corn | 2 | 8 | 0.12 | 17 | 71 | 80 |
| Dent corn | 3 | 8 | 0.21 | 9 | 48 | 62 |
| Dent corn | 4 | 10 | 0.31 | 1 | 8 | 15 |
| Dent corn | 5 | 12 | 0.40 | 0 | 2 | 4 |

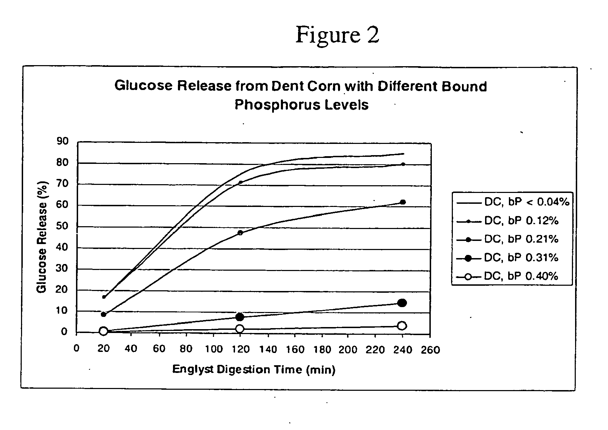

As can be seen from Table II, Sample 3 shows that starch may be crosslinked using a combination of STMP and STPP to result in the altered digestion curve of this invention. The digestion curves of these starches are depicted in Figure 2.

### Example 3 - Glucose Release from Chemically modified Starches

A variety of base starches were modified using PO (not according to the invention), OSA, Acetic Anhydride reagents according to the general procedures described in the above examples to obtain a variety of modification levels. The digestibility of these starches were tested and the results are listed in Table III, below.

**Table III**

| Sample # | Base Starch | Total Bound Phosphorus (%) | Model | T=20 min | T=120 min | T=240 min |
|---|---|---|---|---|---|---|
| | | | | | | |
| 1 | Dent | N/A | N/A | 18 | 80 | 85 |
| 2 | Dent | N/A | Cookie | 29 | 73 | 80 |
| 3 | Dent | 0.24 | N/A | 1 | 27 | 60 |
| 4 | Dent | 0.12 | Cookie | 19 | 65 | 75 |
| 5 | Dent | 0.14 | Cookie | 14 | 47 | 56 |
| 6 | High (~70%) Amylose | N/A | N/A | 11 | 26 | 30 |
| 7 | High (~70%) Amylose | N/A | Cookie | 9 | 23 | 27 |
| 8 | High (~70%) Amylose | 0.23 | N/A | 6 | 13 | 16 |
| 9 | High (~70%) Amylose | 0.25 | Cookie | 7 | 16 | 18 |
| | | | | | | |
| 10 | Tapioca | N/A | N/A | 9 | 42 | 52 |
| 11. | Tapioca | 0.15 | Cookie | 14 | 46 | 58 |
| | | | | | | |
| 12 | Waxy corn | N/A | N/A | 35 | 94 | 100 |
| 13 | Waxy corn | 0.31 | Cookie | 17 | 50 | 60 |
| 14 | Waxy corn | 0.41 | Cookie | 12 | 31 | 36 |
| | | | | | | |
| 15 | Rice | 0.17 | N/A | 17 | 55 | 68 |
| | | | | | | |
| 16 | Wheat | 0.21 | N/A | 22 | 69 | 82 |

As can be seen from Table III, a variety of starch bases may be modified using a combination of STMP and STPP to result in the altered digestion curve of this invention in model food system.

### Example 4 - Glucose Release from Chemically modified Starches

A variety of base starches were modified using PO (not according to the invention), OSA, Acetic Anhydride reagents according to the general procedures described in the above examples to obtain a variety of modification levels. The digestibility of these starches were tested and the results are listed in Table IV, below.

**Table IV**

| Sample # | Sample | Chemical Modifications | | | | Glucose Release at | | |
|---|---|---|---|---|---|---|---|---|
| | Starch Base | Modification 1 | Modification 1 Level | Modification 2 | Modification 2 Level | 20' | 120' | 240' |
| | | | | | | | | |
| 17 | Waxy Corn | na | na | na | na | 35 | 94 | 100 |
| 18 | | OSA | 3% bound OSA | -- | -- | 20 | 56 | 69 |
| 19 | | OSA | 3% bound OSA | Acid Conversion | 25s viscosity | 22 | 69 | 76 |
| 20 | | Acetylation | 2% bound acetyl | Adipic Acidic | 0.55% bound adipate | 23 | 53 | 60 |
| | | | | | | | | |
| 21 | Tapioca | na | na | na | na | 9 | 42 | 52 |
| 22 | | Acetylation | 2% bound acetyl | -- | -- | 19 | 53 | 64 |
| 23 | | Hydroxyprop ylation | 5% bound PO | Phosphoryla tion | 0.04% POCl₃ treatment | 20 | 58 | 66 |
| 24 | | Dextrinizatio n | White, viscosity 5.5 ABF | -- | -- | 21 | 55 | 65 |
| | | | | | | | | |
| 25 | Sago | na | na | na | na | 3 | 13 | 19 |
| 26 | | Hydroxyprop ylation | 7% bound PO | Phosphoryla tion | 0.004% POCl₃ treatment | 21 | 69 | 74 |
| | | | | | | | | |
| 27 | High (~70%) Amylose | na | na | na | na | 11 | 26 | 30 |
| 28 | | OSA | 3% bound OSA | -- | -- | 24 | 64 | 67 |
| 29 | | STMP/STP 99:1 | 0.35% bound P | Bleaching | 0.8% NaOCI | 22 | 56 | 61 |

As can be seen from Table V, a variety of starch bases may be modified using various reagents and/or treatments to result in the altered digestion curve of this invention.

### Example 5 - Food Products Containinq Chemically Modified Starch

The starch samples of the above examples are added at levels of 5-40% to replace flour or other carbohydrate ingredients in six different food products.
1) White Pan Bread
2) Semolina Pasta
3) Nutrition Bar
4) Flavored Yogurt Drink
5) Tea Biscuit
6) Cereals
All ingredients are listed as weight % of the formulation

### 1) White Pan Bread

| | |
|---|---|
| Patent Flour | 55.6 |
| White Granulated Sugar | 4.3 |
| Shortening | 2.8 |
| lodized Salt | 1.1 |
| Active Dry Yeast | 0.6 |
| Dough Conditioner | 35.0 |
| Water | 0.6 |
| Total | 100.0 |

### Preparation:

Combine all ingredients and water in Hobart mixer. Mix on low speed for 2 minutes. Mix on Medium speed for 14 minutes. Allow dough to rest 5 minutes. Scale dough to loaves (510g for ½ kg Loaves). Allow dough to rest 5 minutes. Mold loaves in Glimek Dough-molder. Proof at 90% RH, 80°C. Bake at 210°C for 22 minutes.

### 2) Semolina Pasta

| | |
|---|---|
| Semolina Flour | 74.1 |
| Water | 23.3 |
| Dried Egg Whites | 1.5 |
| Dough Conditioner | 1.1 |
| Total | 100.0 |

### Preparation:

Combine all ingredients and water in Hobart/Kitchen Aid mixer. Mix on low speed for 10 minutes. Feed into sheeter to form into noodles. Cook by placing noodles in boiling water for 5-10 minutes with stirring. Drain water

### 3) Nutrition Bar

| | |
|---|---|
| Protein Powder | 33.6 |
| Brown Rice Syrup | 21.3 |
| Dry Oats | 10.5 |
| Honey | 9.0 |
| Nonfat Dry Milk | 9.7 |
| Soy Oil | 2.8 |
| Peanut Flour | 5.3 |
| Apple Sauce or Raisin Paste | 7.8 |
| Total | 100.0 |

### Preparation:

Combine all dry ingredients (except oats) in Hobart mixer. Mix on low speed for 5 minutes, or until blended. Continue mixing while adding liquid ingredients. Fold in oats while continuing to mix at low speed. Form bar into desired shape by pressing into a form.

### 4) Flavored Yogurt Drink

| | |
|---|---|
| Whole Milk | up to 100.0 |
| Starter culture (Danisco's Jo-mix NM 1-20) | |
| Nonfat Dry Milk | optional |
| Total | 100.0 |

### Yogurt Preparation:

Preheat milk to 65°C. Homogenize at 10.34 megapascal, then hold for 2 minutes at 93°C. Cool mix to 44°C. Inoculate with starter culture. Incubate until pH reaches 4.5 then cool to 4.5°C. Yogurt may be pumped to smooth curd.

**Juice mix:**

| | |
|---|---|
| Water | 47.5 |
| Strawberry conc. (40-60 brix) | 40.0 |
| Fructose | 10.0 |
| Pectin | 2.5 |
| Total | 100.0 |

### Juice Preparation:

Dry blend fructose and pectin. Add dry mix, water, and strawberry concentrate to a blender. Blender until fructose and pectin are dispersed. Cook juice mix in a hot water bath at 80°C for 15 minutes. Cool to 4.5°C.

### Final Product Preparation:

Blend Yogurt and Juice Mix at a ration of 9:1. Co-Homogenize at megapascals of 17.3/3.5 (two stages). Store finished product at 4.5°C

### 5) Tea Biscuit

| | |
|---|---|
| Wheat Flour | 48.0 |
| White Granulated Sugar | 20.5 |
| Whey Powder | 1.3 |
| Baking Powder | 1.2 |
| Salt | 0.6 |
| Shortening | 9.6 |
| Egg Yolks | 2.0 |
| Water | 16.8 |
| Total | 100.0 |

### Preparation:

Combine all dry ingredients and shortening in a Hobart mixer. Mix on low for 5 minutes. Add egg yolks and water. Mix on low for 5 minutes. Roll or sheet dough and cut biscuits. Bake at 176°C for 12-15 minutes.

### 6) Cereal

### a) Extruded breakfast cereal (maize based)

| | |
|---|---|
| Modified maize starch or flour | 40.0% |
| Maize polenta | 45.0% |
| Sugar | 10.0% |
| Salt | 2.0% |
| Malt | 3.0% |
| | 100.0% |

### b) Extruded breakfast cereal (multigrain)

| | |
|---|---|
| Modified maize starch or flour | 43.0% |
| Rice flour | 11.5% |
| Oat flour | 11.5% |
| Wheat flour | 20.4% |
| Sugar | 9.0% |
| Malt | 2.6% |
| Salt | 2.0% |
| | 100.0% |

### Preparation:

The cereals are prepared using methods known in the art. They are extruded, flaked and toasted or extruded and expanded). The cereals are further dried, if necessary, to a final moisture content less than 3%.

The foods are digested using Englyst digestion method and glucose release is monitored over 20, 120 and 240min. The release of glucose is linear over the digestion time.

## Claims

1. Use of starch which is modified by a process selected from the group consisting of propylene oxide modification, octenyl succinic anhydride modification, acetylation, dextrinization, and combinations thereof for the preparation of an edible product for controlling the blood glucose level of a mammal comprising ingesting said product wherein the starch is in granular form and has an altered digestion profile such that less than 25% is digested within the first 20 minutes of ingestion, 30 to 70% is digested within 120 minutes of ingestion and at least 60 % is digested within 240 minutes of ingestion, wherein the digestion profile is measured according to the simulated digestion method of Englyst et al, Eur. J. Clin. Nutr. 46 (1992): S33-S50, as described in the description.

2. Use according to claim 1, wherein the starch provides less than 20% of the glucose release at 20 minutes.

3. Use according to claim 1, wherein the starch provides less than 10% of the glucose release at 20 minutes.

4. Use according to claim 1, wherein the starch provides less than 40 - 60% of the glucose release at 120 minutes.

5. Use according to claim 1, wherein the starch provides less than 45 - 55% of the glucose release at 120 minutes.

6. Use according to claim 1, wherein the starch provides less than 70% of the glucose release at 240 minutes.

7. Use according to claim 1, wherein the starch provides less than 80% of the glucose release at 240 minutes.

8. Use according to claim 1, wherein the starch provides less than 90% of the glucose release at 240 minutes.

9. Use according to claim 1, wherein the starch is present in an amount of 5 - 40% dry weight based on the edible product.

10. Use according to claim 1, wherein the glucose release from the edible product is substantially zero order.

11. Use according to claim 1, wherein the glucose release rate is substantially constant over the first 240 minutes.

12. Use according to claim 1, wherein the starch is modified by a process selected from the group consisting of propylene oxide oxidation in the range of 3 - 10% bound, OSA modification in the range of 1.5 - 3.0% bound, acetylation in the range of 0.5 to 3.0% bound, and combinations thereof.

13. Use according to claim 12, wherein the starch is further modified by a process selected from the group consisting of acid conversion, enzyme conversion, hydrolysis, hypochloride treatment, adipic acetic treatment, phosphorus oxychloride treatment, and combinations thereof.

14. Use according to claim 13, wherein the starch is further modified by a process selected from the group consisting of acid conversion to a water fluidity of 20 - 85, hypochloride treatment at a level of 0.4 - 5.0%, adipic acetic treatment at a level of 0.1 to 2.0%, phosphorus oxychloride treatment at a level of 0.001 to 0.5% treatment, and combinations thereof.

15. Use according to claim 1, wherein the starch is treated with sodium trimetaphosphate and/or sodium tripolyphosphate at a level of 0.1 to 0.35% added bound phosphate and hypochloride treatment at a level of 0.3 to 1.0%.

## Patentansprüche

1. Verwendung von Stärke, die durch ein Verfahren modifiziert ist, das aus der Gruppe, bestehend aus Propylenoxid-Modifikation, Octenylbernsteinsäureanhydrid-Modifikation, Acetylierung, Dextrinierung und Kombinationen davon, ausgewählt ist, für die Herstellung eines essbaren Produkts zur Regulierung des Blutglucosespiegels eines Säugers, umfassend Aufnehmen des Produkts, in dem die Stärke in körniger Form vorliegt und ein verändertes Verdauungsprofil hat, so dass weniger als 25 % innerhalb der ersten 20 Minuten nach Ingestion verdaut werden, 30 bis 70 % innerhalb von 120 Minuten nach Ingestion verdaut werden und wenigstens 60 % innerhalb von 240 Minuten nach Ingestion verdaut werden, wobei das Verdauungsprofil nach dem simulierten Verdauungsverfahren von Englyst et al., Eur. J. Clin. Nutr., 46 (1992) : S33-S50, das in der Beschreibung beschrieben wird, gemessen wird.

2. Verwendung nach Anspruch 1, wobei die Stärke weniger als 20 % der Glucosefreisetzung in 20 Minuten bereitstellt.

3. Verwendung nach Anspruch 1, wobei die Stärke weniger als 10 % der Glucosefreisetzung in 20 Minuten bereitstellt.

4. Verwendung nach Anspruch 1, wobei die Stärke weniger als 40 bis 60 % der Glucosefreisetzung in 120 Minuten bereitstellt.

5. Verwendung nach Anspruch 1, wobei die Stärke weniger als 45 bis 55 % der Glucosefreisetzung in 120 Minuten bereitstellt.

6. Verwendung nach Anspruch 1, wobei die Stärke weniger als 70 % der Glucosefreisetzung in 240 Minuten bereitstellt.

7. Verwendung nach Anspruch 1, wobei die Stärke weniger als 80 % der Glucosefreisetzung in 240 Minuten bereitstellt.

8. Verwendung nach Anspruch 1, wobei die Stärke weniger als 90 % der Glucosefreisetzung in 240 Minuten bereitstellt.

9. Verwendung nach Anspruch 1, wobei die Stärke in einer Menge von 5 bis 40 % Trockengewicht, basierend auf dem essbaren Produkt, vorliegt.

10. Verwendung nach Anspruch 1, wobei die Glucosefreisetzung aus dem essbaren Produkt im Wesentlichen nullter Ordnung ist.

11. Verwendung nach Anspruch 1, wobei die Glucosefreisetzungsrate über die ersten 240 Minuten im Wesentlichen konstant ist.

12. Verwendung nach Anspruch 1, wobei die Stärke durch ein Verfahren modifiziert ist, das ausgewählt ist aus der Gruppe, bestehend aus Propylenoxidoxidation im Bereich von gebundenen 3 bis 10 %, OSA-Modifikation im Bereich von gebundenen 1,5 bis 3,0 % Acetylierung im Bereich von gebundenen 0,5 bis 3,0 % und Kombinationen davon.

13. Verwendung nach Anspruch 12, wobei die Stärke außerdem durch ein Verfahren modifiziert ist, das aus der Gruppe, bestehend aus Säureumwandlung, Enzymumwandlung, Hydrolyse, Hypochloritumwandlung, Adipinessigsäurebehandlung, Phosphoroxychloridbehandlung und Kombinationen davon, ausgewählt ist.

14. Verwendung nach Anspruch 13, wobei die Stärke außerdem durch ein Verfahren modifiziert ist, das aus der Gruppe, bestehend aus Säureumwandlung zu einer Wasserfluidität von 20 bis 85, Hypochloritbehandlung in einer Konzentration von 0,4 bis 5,0 %, Adipinessigsäurebehandlung in einer Konzentration von 0,1 bis 2,0 %, Phosphoroxychloridbehandlung in einer Konzentration von 0,001 bis 0,5 % Behandlung und Kombinationen davon ausgewählt ist.

15. Verwendung nach Anspruch 1, wobei die Stärke mit Natriumtrimetaphosphat und/oder Natriumtripolyphosphat in einer Konzentration von 0,1 bis 0,35 % addiertes gebundenes Phosphat und Hypochloritbehandlung in einer Konzentration von 0,3 bis 1,0 % behandelt ist.

## Revendications

1. Utilisation d'un amidon qui est modifié par un procédé choisi dans le groupe consistant en une modification avec de l'oxyde de propylène, une modification avec de l'anhydride octénylsuccinique, une acétylation, une dextrinisation et leurs associations pour la préparation d'un produit comestible afin de réguler le taux sanguin de glucose d'un mammifère, comprenant l'ingestion dudit produit dans lequel l'amidon est sous forme granulaire et a un profil de digestion modifié tel qu'une proportion inférieure à 25 % soit digérée au cours des 20 premières minutes de l'ingestion, une proportion de 30 à 70 % soit digérée au cours de 120 minutes d'ingestion, une proportion d'au moins 60 % soit digérée en moins de 240 minutes d'ingestion, dans laquelle le profil de digestion est mesuré suivant la méthode de digestion simulée d'Englyst et al., Eur. J. Clin. Nutr. 46 (1992): S33-S50, comme décrit dans la description.

2. Utilisation suivant la revendication 1, dans laquelle l'amidon présente une libération de moins de 20 % du glucose à 20 minutes.

3. Utilisation suivant la revendication 1, dans laquelle l'amidon présente une libération de moins de 10 % du glucose à 20 minutes.

4. Utilisation suivant la revendication 1, dans laquelle l'amidon présente une libération de moins de 40-60 % du glucose à 120 minutes.

5. Utilisation suivant la revendication 1, dans laquelle l'amidon présente une libération de moins de 45-55 % du glucose à 120 minutes.

6. Utilisation suivant la revendication 1, dans laquelle l'amidon présente une libération de moins de 70 % du glucose à 240 minutes.

7. Utilisation suivant la revendication 1, dans laquelle l'amidon présente une libération de moins de 80 % du glucose à 240 minutes.

8. Utilisation suivant la revendication 1, dans laquelle l'amidon présente une libération de moins de 90 % du glucose à 240 minutes.

9. Utilisation suivant la revendication 1, dans laquelle l'amidon est présent en une quantité de 5 à 40 % de poids sec sur la base du produit comestible.

10. Utilisation suivant la revendication 1, dans laquelle la libération du glucose par le produit comestible est pratiquement d'ordre zéro.

11. Utilisation suivant la revendication 1, dans laquelle la vitesse de libération du glucose est pratiquement constante au cours des 240 premières minutes.

12. Utilisation suivant la revendication 1, dans laquelle l'amidon est modifié par un procédé choisi dans le groupe consistant en une oxydation avec de l'oxyde de propylène en une quantité liée comprise dans l'intervalle de 3 à 10 %, une modification avec du OSA en une quantité liée comprise dans l'intervalle de 1,5 à 3,0 %, une acétylation en une quantité liée comprise dans l'intervalle de 0,5 à 3,0 % et leurs associations.

13. Utilisation suivant la revendication 12, dans laquelle l'amidon est en outre modifié par un procédé choisi dans le groupe consistant en une saccharification avec un acide, une saccharification enzymatique, une hydrolyse, un traitement avec un hypochlorite, un traitement adipique-acétique, un traitement avec de l'oxychlorure de phosphore et leurs associations.

14. Utilisation suivant la revendication 13, dans laquelle l'amidon est en outre modifié par un procédé choisi dans le groupe consistant en une saccharification avec un acide jusqu'à une fluidité en milieu aqueux de 20 à 85, un traitement avec un hypochlorite à un taux de 0,4 à 5,0 %, un traitement adipique-acêtique à un taux de 0,1 à 2,0 %, un traitement avec de l'oxychlorure de phosphore à un taux de 0,001 à 0,5 % et leurs associations.

15. Utilisation suivant la revendication 1, dans laquelle l'amidon est traité avec du trimétaphosphate de sodium et/ou du tripolyphosphate de sodium à un taux de 0,1 à 0,35 % de phosphate lié ajouté et est traité avec un hypochlorite à un taux de 0,3 à 1,0 %.
